(19) Europäisches Patentamt / European Patent Office / Office européen des brevets

(11) **EP 4 629 167 A1**

(12) **EUROPEAN PATENT APPLICATION**
published in accordance with Art. 153(4) EPC

(43) Date of publication:
**08.10.2025 Bulletin 2025/41**

(21) Application number: **23896940.6**

(22) Date of filing: **01.12.2023**

(51) International Patent Classification (IPC):
**G06T 7/00** (2017.01)  **G06T 5/50** (2006.01)

(86) International application number:
**PCT/CN2023/135877**

(87) International publication number:
**WO 2024/114798 (06.06.2024 Gazette 2024/23)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **01.12.2022 CN 202211532207**

(71) Applicant: **Nanovision Medical Technology
(Shanghai) Co., Ltd.
Shanghai 201318 (CN)**

(72) Inventor: **DING, Haining
Shanghai 201318 (CN)**

(74) Representative: **Wang, Bo
Panovision IP
Ebersberger Straße 3
85570 Markt Schwaben (DE)**

(54) **BLOOD VESSEL RECOGNITION MONITORING METHOD AND SYSTEM BASED ON STATIC CT ENHANCEMENT SCANNING**

(57)    Disclosed in the present invention are a blood vessel recognition monitoring method and system based on static CT enhancement scanning. The method comprises the following steps: acquiring subtraction images of all regions of a blood vessel to be monitored of a patient; performing image processing on the subtraction images to equally divide each subtraction image into a plurality of region blocks of a preset size; calculating a mean value and a standard deviation for each region block of each subtraction image, and calculating P values of the region blocks; arranging the P values of the same region blocks of the subtraction images in chronological order, and drawing a P-value change curve of the region blocks; and drawing a concentration change curve of a contrast agent on the basis of the P-value change curve of the region blocks to determine the time to peak of the contrast agent.

```
S1: Obtain images
        |
        v
S2: Process images
        |
        v
S3: Calculate a P value of each region block in
    each subtraction image
        |
        v
S4: Plot a P value change curve of the region
    block
        |
        v
S5: Determine the time to peak of a contrast
    agent
```

FIG. 1

EP 4 629 167 A1

**Description**

**BACKGROUND**

**Technical Field**

The present disclosure relates to a blood vessel recognition monitoring method based on static CT enhancement scanning, and also relates to a blood vessel recognition monitoring system configured to implement the blood vessel recognition monitoring method, belonging to the technical field of medical images.

**Related Art**

The augmented scanning is to increase the density difference between an affected tissue and an adjacent normal tissue, thereby increasing the occurrence rate of a focus. When the augmented scanning is performed, there is individual difference for different patients, and times to peak of contrast agents in monitored blood vessels are inconsistent. If a doctor directly sets the time to peak by using an empirical value, an optimal time for third-phase scanning is easily missed. A small dose augmented scanning mode needs to be used to obtain the time to peak of a contrast agent in a blood vessel to be monitored.

A scanning process for small dose augmented scanning for monitoring of a blood vessel by means of CT is to first perform CT scanning. A doctor delineates a region of interest (ROI) of a monitored blood vessel on a tomographic image, injects a small dose of contrast agent to a patient for the first time, monitors a curve of change of the concentration of the contrast agent of the blood vessel to be monitored with time, and determines the time to peak of the contrast agent. The doctor sets a third-phase scanning time according to the time to peak of the first small dose scanning, and injects a small dose of contrast agent for the second time to the patient to complete the subsequent task of third-phase scanning. When the position of the ROI of the blood vessel to be monitored delineated by the doctor deviates from the original blood vessel, the change of the contrast agent in the blood vessel to be monitored is inaccurate, and the obtained curve naturally cannot well reflect the change of the concentration of the contrast agent with time.

**SUMMARY**

The first technical problem to be solved by the present disclosure is to provide a blood vessel recognition monitoring method based on static CT enhancement scanning.

Another technical problem to be solved by the present disclosure is to provide a blood vessel recognition monitoring system based on static CT enhancement scanning.

In order to achieve the foregoing objectives, the present disclosure adopts the following technical solutions: According to a first aspect of embodiments of the present disclosure, a blood vessel recognition monitoring method based on static CT enhancement scanning is provided, including the following steps:

> obtaining subtraction images of all regions of a blood vessel to be monitored of a patient;
> performing image processing on the subtraction images so that each subtraction image is evenly divided into a plurality of region blocks having a preset size, and the positions of the region blocks of each subtraction image are in one-to-one correspondence;
> calculating a mean value and a standard deviation of each region block of each subtraction image, and calculating a P value of the region block;
> arranging the P value of the same region block of each subtraction image according to a time sequence, and plotting a P value change curve of the region block; and
> plotting a contrast agent concentration change curve of the region block based on the P value change curve of the region block, so as to determine the time to peak of a contrast agent.

Preferably, the obtaining subtraction images of all regions of a blood vessel to be monitored of a patient specifically includes:

> projecting all regions of the blood vessel to be monitored of the patient in a state in which the patient is injected with a small dose of contrast agent;
> obtaining an image of all regions at an interval of every preset time within a preset duration;
> subtracting a first image of all regions from an obtained second image of all regions to obtain a first subtraction image;
> subtracting the first image of all regions from an obtained third image of all regions to obtain a second subtraction image; subtracting the first image of all regions from an obtained fourth image of all regions to obtain a third subtraction image; and repeating the processes to obtain a last subtraction image,
where the first subtraction image to the last subtraction image jointly constitute the subtraction images.

Preferably, the preset size of the region block is 16*16 pixels, so as to match the diameter of the blood vessel to be monitored.

Preferably, the calculating a mean value and a standard deviation of each region block of each subtraction image, and calculating a P value of the region block specifically includes:

calculating a mean value $\mu$ of the region block based on the following formula:

$$\mu = \frac{1}{MN}\sum_{i=1}^{M}\sum_{j=1}^{N} H_{i,j}$$

calculating a standard deviation $\sigma$ of the region block based on the following formula:

$$\sigma = \sqrt{\frac{1}{MN}\sum_{i=1}^{M}\sum_{j=1}^{N}\left(H_{i,j} - \mu\right)^2}$$

and calculating a P value of the region block according to the mean value $\mu$ and the standard deviation $\sigma$ based on the following formula:

$$p = \frac{\mu_{n-}(\mu_0 + \sigma_0)}{\sigma_0}$$

where M and N represent pixel numbers of an image; $H_{i,j}$ represents the values of a pixel point (i, j); $\mu_0$ represents a mean value at time $t_0$, and $\sigma_0$ represents a standard deviation at time $t_0$, that is, the mean value and the standard deviation of the first image; and $\mu_n$ represents a mean value at time $t_n$, that is, a mean value of an (n-1)$^{th}$ image.

Preferably, the plotting a contrast agent concentration change curve of the region block based on the P value change curve of the region block, so as to determine the time to peak of a contrast agent specifically includes:

monitoring a change in the mean value of the region block according to the P value change curve of the region block to obtain a concentration change trend of the contrast agent;
plotting a concentration change curve of the contrast agent based on the concentration change trend of the contrast agent; and
obtaining the corresponding time when the concentration of the contrast agent reaches a peak value based on the concentration change curve of the contrast agent, that is, the time to peak of the contrast agent.

Preferably, the blood vessel recognition monitoring method further includes:
stopping CT scanning of the patient based on the concentration change curve of the contrast agent when the concentration of the contrast agent starts to decrease from the peak value.

According to a second aspect of embodiments of the present disclosure, a blood vessel recognition monitoring system based on static CT enhancement scanning is provided, including:

an image obtaining unit, configured to obtain subtraction images of a blood vessel to be monitored of a patient;
an image processing unit, connected to the image obtaining unit, and configured to perform image processing on the subtraction images so that each subtraction image is evenly divided into a plurality of region blocks having a preset size, and the positions of the region blocks of each subtraction image are in one-to-one correspondence;
a calculation unit, connected to the image processing unit to calculate a P value of the region block of the processed subtraction image; and
an editing unit, connected to the calculation unit to plot a contrast agent concentration change curve of the region block, so as to determine the time to peak of a contrast agent.

Compared with the prior art, the present disclosure has the following technical effects:

1. The position and change trend of the blood vessel to be monitored can be accurately determined to avoid the situation that the position of the ROI of the blood vessel to be monitored delineated by a doctor deviates from the original blood vessel, consequently, the monitored CT value of the blood vessel is inaccurate, therefore, a change curve of the CT value with time cannot be well reflected, and the time to peak of the contrast agent is missed.

2. By observing the contrast agent concentration change curve, if a decrease trend occurs, the CT scanning can be terminated at any time to prevent a patient from receiving unnecessary X-rays, thereby reducing the radiation dose received by the patient.

**BRIEF DESCRIPTION OF THE DRAWINGS**

FIG. 1 is a flowchart of a blood vessel recognition monitoring method based on static CT enhancement scanning according to a first embodiment of the present disclosure;

FIG. 2 is a schematic structural diagram of performing region block division on a subtraction image according to the first embodiment of the present disclosure;

FIG. 3 is a structural diagram of a blood vessel recognition monitoring system based on static CT enhancement scanning according to a second embodiment of the present disclosure; and

FIG. 4 is a structural diagram of a blood vessel recognition monitoring system based on static CT enhancement scanning according to a third embodiment of the present disclosure.

**DETAILED DESCRIPTION**

The technical content of the present disclosure is specifically described in detail below with reference to accompanying drawings and specific embodiments.

First embodiment

First, it should be noted that the blood vessel recognition monitoring method provided in this embodiment of the present disclosure is mainly for second-phase scanning, that is, after a small dose of contrast agent is injected to a patient for the first time, the time to peak of the contrast agent is accurately determined to avoid missing an optimal scanning occasion during third-phase scanning.

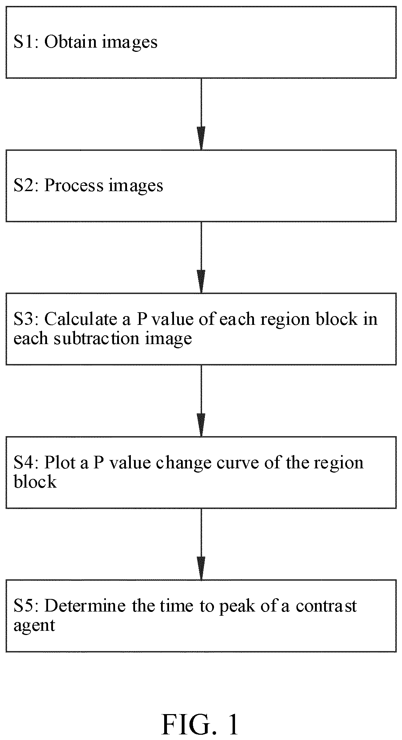

As shown in FIG. 1, a first embodiment of the present disclosure provides a blood vessel recognition monitoring method based on static CT enhancement scanning, specifically including steps S1 to S5.

S1: Images are obtained.

Specifically, steps S11 to S13 are included.

S11: All regions of a blood vessel to be monitored of a patient are projected in a state in which the patient is injected with a small dose of contrast agent.

It can be understood that, in this embodiment, not an ROI in which a doctor delineates a monitored blood vessel on a tomographic image is monitored, but all regions of the monitored blood vessel are monitored, thereby ensuring the comprehensiveness of monitoring results.

S12: An image of all regions is obtained at an interval of every preset time within a preset duration.

Specifically, in this embodiment, an image of all regions is obtained every 250 ms, and 51 images of all regions are obtained in total.

S13: Subtraction images are obtained.

Specifically, a first image of all regions is subtracted from an obtained second image of all regions to obtain a first subtraction image; the first image of all regions is subtracted from an obtained third image of all regions to obtain a second subtraction image; and the processes are repeated to obtain a last subtraction image. Therefore, 50 subtraction images can be obtained by using 51 images of all regions, and the 50 subtraction images jointly constitute a group of subtraction images.

S2: Images are processed.

Specifically, in this embodiment, after the 50 subtraction images are obtained in step S1, image processing needs to be performed on each subtraction image, so that each subtraction image is evenly divided into a plurality of region blocks having a preset size, and the positions of the region blocks of the 50 subtraction images are in one-to-one correspondence.

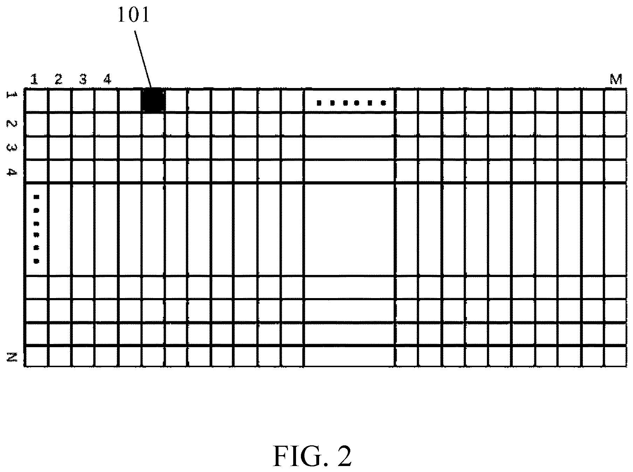

Referring to FIG. 2, in this embodiment, each subtraction image includes m*n (both m and n are positive integers and are the same below) pixel blocks. The m*n pixel blocks are divided to form a plurality of region blocks 101 (shown as black regions in FIG. 2), and each region block 101 includes a*a pixel blocks. Because the sizes of the subtraction images are the same, the quantities of the divided region blocks 101 are also the same. Therefore, the positions of the region blocks of the 50 subtraction images are in one-to-one correspondence.

In one embodiment of the present disclosure, preferably, the region block 101 includes 16*16 pixel blocks, where each pixel block is approximately 0.265 mm, and the size of the 16* 16 pixel blocks is equivalent to the thickness of the blood vessel to be monitored, so as to match the blood vessel to be monitored, thereby improving the monitoring effect. It can be understood that, in other embodiments, the size of the region block 101 can be adaptively adjusted as required.

S3: A P value of each region block in each subtraction image is calculated.

In this embodiment, after image processing is performed on each subtraction image to divide the plurality of region blocks 101, a P value of each region block 101 needs to be calculated. If a contrast agent appears in a certain region block 101 in the subtraction image, the P value of the region block 101 presents significance, so that the position of the region block 101 can be accurately determined as the position where the contrast agent is located. On the contrary, if no contrast agent appears in the region block in the subtraction image, the P value of the region block 101 does not present significance.

It can be understood that, in this embodiment, P values of a plurality of region blocks 101 need to be calculated for each subtraction image. Whether a P value of any region block 101 presents significance needs to be obtained by comparison with a P value of a region block at a same position in a previous subtraction image. Therefore, the P value at the same position has 50 values as time changes, so that a curve can be plotted, and the significant change indicates the position of the contrast agent.

The calculating a P value of a region block 101 specifically includes steps S31 to S33.

S31: A mean value $\mu$ of the region block 101 is calculated based on Formula (1):

$$\mu = \frac{1}{MN} \sum_{i=1}^{M} \sum_{j=1}^{N} H_{i,j} \tag{1}$$

S32: A standard deviation $\sigma$ of the region block 101 is calculated based on Formula (2):

$$\sigma = \sqrt{\frac{1}{MN} \sum_{i=1}^{M} \sum_{j=1}^{N} \left( H_{i,j} - \mu \right)^2} \tag{2}$$

S33: AP value of the region block 101 is calculated according to the mean value $\mu$ and the standard deviation $\sigma$ based on Formula (3):

$$p = \frac{\mu_{n-}(\mu_0 + \sigma_0)}{\sigma_0} \tag{3}$$

where M and N represent pixel numbers of an image; $H_{i,j}$ represents the values of a pixel point (i, j); $\mu_0$ represents a mean value at time $t_0$, and $\sigma_0$ represents a standard deviation at time $t_0$, that is, the mean value and the standard deviation of the first image; and $\mu_n$ represents a mean value at time $t_n$, that is, a mean value of an (n-1)th image.

S4: A P value change curve of the region block 101 is plotted.

Specifically, after P values of a plurality of region blocks in each subtraction image are calculated based on step S3, the P value of the same region block in each subtraction image is arranged according to a time sequence, thereby determining which region blocks 101 have significant P values and which region blocks 101 have no significant P values, so as to obtain a P value change process, and then plot a P value change curve of the region blocks.

S5: The time to peak of the contrast agent is determined.

Specifically, steps S51 to S53 are included.

S51: A change in the mean value of the region block is monitored according to the P value change curve of the region block to obtain a concentration change trend of the contrast agent.

S52: A concentration change curve of the contrast agent is plotted based on the concentration change trend of the contrast agent.

S53: The corresponding time is obtained when the concentration of the contrast agent reaches a peak value based on the concentration change curve of the contrast agent, that is, the time to peak of the contrast agent.

Therefore, the blood vessel recognition monitoring method provided in the embodiments of the present disclosure can accurately determine the position and change trend of the blood vessel to be monitored to avoid the situation that the position of the ROI of the blood vessel to be monitored delineated by a doctor deviates from the original blood vessel, consequently, the monitored CT value of the blood vessel is inaccurate, therefore, a change curve of the CT value with time cannot be well reflected, and the time to peak of the contrast agent is missed. Moreover, in this embodiment, by observing the contrast agent concentration change curve, if a decrease trend occurs, the doctor can terminate CT scanning at any time to prevent a patient from receiving unnecessary X-rays, thereby reducing the radiation dose received by the patient.

Second embodiment

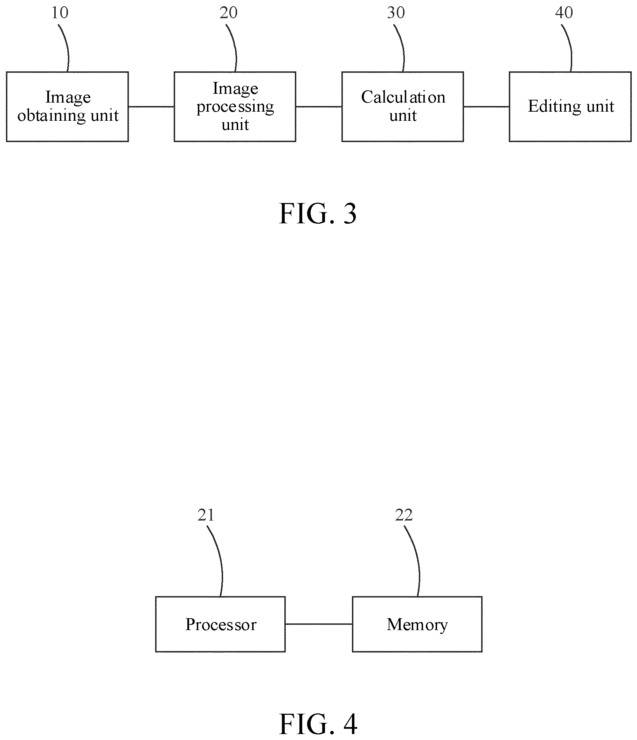

As shown in FIG. 3, based on the foregoing first embodiment, a second embodiment of the present disclosure further provides a blood vessel recognition monitoring system based on static CT enhancement scanning, including at least an image obtaining unit 10, an image processing unit 20, a calculation unit 30, and an editing unit 40.

Specifically, the image obtaining unit 10 is configured to obtain a group of subtraction images of a blood vessel to be monitored of a patient. The image processing unit 20 is connected to the image obtaining unit 10, and configured to perform image processing on the group of subtraction images so that each subtraction image is evenly divided into a plurality of region blocks having a preset size, and the positions of the region blocks of each subtraction image are in one-to-one correspondence. The calculation unit 30 is connected to the image processing unit 20 to calculate a P value of the region block of the processed subtraction image. The editing unit 40 is connected to the calculation unit 30 to plot a contrast agent concentration change curve of the region block, so as to determine the time to peak of a contrast agent.

Third embodiment

As shown in FIG. 4, based on the foregoing first embodiment, a third embodiment of the present disclosure further provides a blood vessel recognition monitoring system based on static CT enhancement scanning, including one or more processors 21 and a memory 22. The memory 22 is coupled to the processor 21 and configured to store one or more programs. When the one or more programs are executed by the one or more processors 21, the one or more processors 21 implement the blood vessel recognition monitoring method as described in the foregoing first embodiment.

The processor 21 is configured to control overall operations of the monitoring system to complete all or some steps of the foregoing blood vessel recognition monitoring method. The processor 21 may be a central processing unit (CPU), a graphics processing unit (GPU), a field programmable gate array (FPGA), an application-specific integrated circuit (ASIC), a digital signal processing (DSP) chip, or the like. The memory 22 is configured to store various types of data to support operations in the monitoring system. The data may include, for example, an instruction of any application program or method used to operate in the monitoring system, and data related to an application program. The memory 22 may be implemented by any type of volatile or non-volatile storage device or a combination thereof, such as a static random access memory (SRAM), an electrically erasable programmable read-only memory (EEPROM), an erasable programmable read-only memory (EPROM), a programmable read-only memory (PROM), a read-only memory (ROM), a magnetic memory, or a flash memory.

In an exemplary embodiment, the monitoring system may be specifically implemented by a computer chip or an entity, or implemented by a product having a function, and is configured to perform the foregoing blood vessel recognition monitoring method, and achieve the technical effects consistent with the foregoing method. A typical embodiment is a computer. Specifically, the computer may be a personal computer, a laptop computer, a vehicle-mounted human-computer interaction device, a cellular phone, a camera phone, a smart phone, a personal digital assistant, a media player, a navigation device, an email device, a game console, a tablet computer, a wearable device, or any combination of these devices.

In another exemplary embodiment, the present disclosure further provides a computer-readable storage medium including a program instruction. The program instruction, when executed by the processor, implements the steps of the blood vessel recognition monitoring method in any of the foregoing embodiments. For example, the computer-readable storage medium may be the foregoing memory including a program instruction, and the foregoing program instruction may be executed by a processor of a monitoring system to complete the foregoing blood vessel recognition monitoring method, and achieve the technical effects consistent with the foregoing method.

The foregoing describes the blood vessel recognition monitoring method and system based on static CT enhancement scanning provided in the present disclosure in detail. Any obvious change made by those of ordinary skill in the art to the present disclosure without departing from the essence of the present disclosure shall constitute a violation of the patent right of the present disclosure and shall take the corresponding legal responsibility.

**Claims**

1. A blood vessel recognition monitoring method based on static CT enhancement scanning, comprising the following steps:

   obtaining subtraction images of all regions of a blood vessel to be monitored of a patient;
   performing image processing on the subtraction images so that each subtraction image is evenly divided into a plurality of region blocks having a preset size, and the positions of the region blocks of each subtraction image are in one-to-one correspondence;

calculating a mean value and a standard deviation of each region block of each subtraction image, and calculating a P value of the region block;

arranging the P value of the same region block of each subtraction image according to a time sequence, and plotting a P value change curve of the region block; and

plotting a contrast agent concentration change curve of the region block based on the P value change curve of the region block, so as to determine the time to peak of a contrast agent.

2. The blood vessel recognition monitoring method according to claim 1, wherein the obtaining subtraction images of all regions of a blood vessel to be monitored of a patient specifically comprises:

projecting all regions of the blood vessel to be monitored of the patient in a state in which the patient is injected with a small dose of contrast agent;

obtaining an image of all regions at an interval of every preset time within a preset duration;

subtracting a first image of all regions from an obtained second image of all regions to obtain a first subtraction image; subtracting the first image of all regions from an obtained third image of all regions to obtain a second subtraction image; and repeating the processes to obtain a last subtraction image,

wherein the first subtraction image to the last subtraction image jointly constitute a group of subtraction images.

3. The blood vessel recognition monitoring method according to claim 1, wherein
the preset size of the region block is 16* 16 pixels, so as to match a diameter of the blood vessel to be monitored.

4. The blood vessel recognition monitoring method according to claim 1, wherein the calculating a mean value and a standard deviation of each region block of each subtraction image, and calculating a P value of the region block specifically comprises:

calculating a mean value $\mu$ of the region block based on the following formula:

$$\mu = \frac{1}{MN} \sum_{i=1}^{M} \sum_{j=1}^{N} H_{i,j}$$

calculating a standard deviation $\sigma$ of the region block based on the following formula:

$$\sigma = \sqrt{\frac{1}{MN} \sum_{i=1}^{M} \sum_{j=1}^{N} \left( H_{i,j} - \mu \right)^2}$$

and calculating a P value of the region block according to the mean value $\mu$ and the standard deviation $\sigma$ based on the following formula:

$$p = \frac{\mu_{n-}(\mu_0 + \sigma_0)}{\sigma_0}$$

wherein M and N represent pixel numbers of an image; $H_{i,j}$ represents the values of a pixel point (i, j); $\mu_0$ represents a mean value at time $t_0$, and $\sigma_0$ represents a standard deviation at time $t_0$, that is, the mean value and the standard deviation of the first image; and $\mu_n$ represents a mean value at time $t_n$, that is, a mean value of an (n-1)th image.

5. The blood vessel recognition monitoring method according to claim 1, wherein the plotting a contrast agent concentration change curve of the region block based on the P value change curve of the region block, so as to determine the time to peak of a contrast agent specifically comprises:

monitoring a change in the mean value of the region block according to the P value change curve of the region block to obtain a concentration change trend of the contrast agent;

plotting a concentration change curve of the contrast agent based on the concentration change trend of the contrast agent; and

obtaining the corresponding time when the concentration of the contrast agent reaches a peak value based on the concentration change curve of the contrast agent, that is, the time to peak of the contrast agent.

6. The blood vessel recognition monitoring method according to claim 1, further comprising:
stopping CT scanning of the patient based on the concentration change curve of the contrast agent when the concentration of the contrast agent starts to decrease from the peak value.

7. A blood vessel recognition monitoring system based on static CT enhancement scanning, comprising:

an image obtaining unit, configured to obtain subtraction images of a blood vessel to be monitored of a patient;
an image processing unit, connected to the image obtaining unit, and configured to perform image processing on the subtraction images so that each subtraction image is evenly divided into a plurality of region blocks having a preset size, and the positions of the region blocks of each subtraction image are in one-to-one correspondence;
a calculation unit, connected to the image processing unit to calculate a P value of the region block of the processed subtraction image; and
an editing unit, connected to the calculation unit to plot a contrast agent concentration change curve of the region block, so as to determine the time to peak of a contrast agent.

8. The blood vessel recognition monitoring system according to claim 7, wherein the obtaining subtraction images of a blood vessel to be monitored of a patient specifically comprises:

projecting all regions of the blood vessel to be monitored of the patient in a state in which the patient is injected with a small dose of contrast agent;
obtaining an image of all regions at an interval of every preset time within a preset duration;
subtracting a first image of all regions from an obtained second image of all regions to obtain a first subtraction image; subtracting the first image of all regions from an obtained third image of all regions to obtain a second subtraction image; and repeating the processes to obtain a last subtraction image,
wherein the first subtraction image to the last subtraction image jointly constitute a group of subtraction images.

9. The blood vessel recognition monitoring system according to claim 8, wherein the calculating a P value of the region block of the processed subtraction image specifically comprises:

calculating a mean value $\mu$ of the region block based on the following formula:

$$\mu = \frac{1}{MN} \sum_{i=1}^{M} \sum_{j=1}^{N} H_{i,j}$$

calculating a standard deviation $\sigma$ of the region block based on the following formula:

$$\sigma = \sqrt{\frac{1}{MN} \sum_{i=1}^{M} \sum_{j=1}^{N} \left(H_{i,j} - \mu\right)^2}$$

and calculating a P value of the region block according to the mean value $\mu$ and the standard deviation $\sigma$ based on the following formula:

$$p = \frac{\mu_{n-}(\mu_0 + \sigma_0)}{\sigma_0}$$

wherein M and N represent pixel numbers of an image; $H_{i,j}$ represents the values of a pixel point (i, j); $\mu_0$ represents a mean value at time $t_0$, and $\sigma_0$ represents a standard deviation at time $t_0$, that is, the mean value and the standard deviation of the first image; and $\mu_n$ represents a mean value at time $t_n$, that is, a mean value of an (n-1)$^{th}$ image.

10. The blood vessel recognition monitoring system according to claim 9, wherein the plotting a contrast agent concentration change curve of the region block, so as to determine the time to peak of a contrast agent specifically comprises:

monitoring a change in the mean value of the region block according to the P value change curve of the region block to obtain a concentration change trend of the contrast agent;
plotting a concentration change curve of the contrast agent based on the concentration change trend of the contrast agent; and

obtaining the corresponding time when the concentration of the contrast agent reaches a peak value based on the concentration change curve of the contrast agent, that is, the time to peak of the contrast agent.

S1: Obtain images

↓

S2: Process images

↓

S3: Calculate a P value of each region block in each subtraction image

↓

S4: Plot a P value change curve of the region block

↓

S5: Determine the time to peak of a contrast agent

FIG. 1

FIG. 2

10       20       30       40

| Image obtaining unit | Image processing unit | Calculation unit | Editing unit |
|---|---|---|---|

## FIG. 3

21       22

| Processor | Memory |
|---|---|

## FIG. 4

## INTERNATIONAL SEARCH REPORT

| International application No. |
| --- |
| **PCT/CN2023/135877** |

**A.    CLASSIFICATION OF SUBJECT MATTER**

G06T7/00(2017.01)i;  G06T5/50(2006.01)i

According to International Patent Classification (IPC) or to both national classification and IPC

**B.    FIELDS SEARCHED**

Minimum documentation searched (classification system followed by classification symbols)

IPC:G06T

Documentation searched other than minimum documentation to the extent that such documents are included in the fields searched

Electronic data base consulted during the international search (name of data base and, where practicable, search terms used)

CJFD, CNTXT, ENTXTC: P值, 标准差, 方差, 分割, 分块, 分区, 划分, 剪影, 减影, 均分, 均值, 浓度, 平均, 图像, 造影剂, 子块, 子区域, CT, DSA, P value, standard deviation, variance, split, sub region, sub block, divid+, silhouette, subtract, mean, average, concentration, image, contrast agent

**C.    DOCUMENTS CONSIDERED TO BE RELEVANT**

| Category* | Citation of document, with indication, where appropriate, of the relevant passages | Relevant to claim No. |
| --- | --- | --- |
| PX | CN 116309267 A (NAMI WEIJING (SHANGHAI) MEDICAL TECHNOLOGY CO., LTD.) 23 June 2023 (2023-06-23) <br> claims 1-10 | 1-10 |
| A | US 2021259654 A1 (SHANGHAI UNITED IMAGING HEALTHCARE CO., LTD.) 26 August 2021 (2021-08-26) <br> description, paragraphs [0073]-[0096] | 1-10 |
| A | CN 111789625 A (NEUSOFT MEDICAL SYSTEMS CO., LTD.) 20 October 2020 (2020-10-20) <br> entire document | 1-10 |
| A | CN 112053414 A (ZHEJIANG UNIVERSITY) 08 December 2020 (2020-12-08) <br> entire document | 1-10 |
| A | CN 101127117 A (HUAZHONG UNIVERSITY OF SCIENCE AND TECHNOLOGY) 20 February 2008 (2008-02-20) <br> entire document | 1-10 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **04 February 2024** | **18 February 2024** |

| Name and mailing address of the ISA/CN | Authorized officer |
| --- | --- |
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| | International application No. |
|---|---|
| | **PCT/CN2023/135877** |

### C. DOCUMENTS CONSIDERED TO BE RELEVANT

| Category* | Citation of document, with indication, where appropriate, of the relevant passages | Relevant to claim No. |
|---|---|---|
| A | US 2016089100 A1 (SIEMENS AKTIENGESELLSCHAFT) 31 March 2016 (2016-03-31) entire document | 1-10 |

Form PCT/ISA/210 (second sheet) (July 2022)

**INTERNATIONAL SEARCH REPORT**
Information on patent family members

| International application No. |
| PCT/CN2023/135877 |

| Patent document cited in search report | | | Publication date (day/month/year) | Patent family member(s) | | | Publication date (day/month/year) |
|---|---|---|---|---|---|---|---|
| CN | 116309267 | A | 23 June 2023 | None | | | |
| US | 2021259654 | A1 | 26 August 2021 | EP | 3869460 | A1 | 25 August 2021 |
| | | | | CN | 111369588 | A | 03 July 2020 |
| CN | 111789625 | A | 20 October 2020 | None | | | |
| CN | 112053414 | A | 08 December 2020 | None | | | |
| CN | 101127117 | A | 20 February 2008 | None | | | |
| US | 2016089100 | A1 | 31 March 2016 | US | 11020081 | B2 | 01 June 2021 |
| | | | | DE | 102013210613 | A1 | 11 December 2014 |
| | | | | WO | 2014195077 | A1 | 11 December 2014 |

Form PCT/ISA/210 (patent family annex) (July 2022)